# EUROPÄISCHE PATENTSCHRIFT

(11) **EP 3 087 902 B1**
(45) Veröffentlichungstag und Bekanntmachung des Hinweises auf die Patenterteilung: **03.07.2019**
(21) Anmeldenummer: 16167268.8
(22) Anmeldetag: 27.04.2016
(51) Int. Cl.: A61B 1/00, A61M 16/04, A61B 1/005, A61B 1/05, A61B 1/06, A61B 1/01, A61B 1/267

(54) **VIDEOENDOSKOP**
VIDEO ENDOSCOPE
ENDOSCOPE VIDEO

(30) Priorität: 29.04.2015 DE 102015106609
(43) Veröffentlichungstag der Anmeldung: 02.11.2016
(73) Patentinhaber: Karl Storz SE & Co. KG, 78532 Tuttlingen (DE)
(72) Erfinder: BRAMBRINK, Ansgar, 97239 Portland (US); HUBER, Christian, 8200 Schaffhausen (CH); HÜLS, Dieter, 78333 Stockach (DE); RUEGG, Thomas, 8200 Schaffhausen (CH); EFINGER, Andreas, 78532 Tuttlingen (DE); RENNER, Martin, 78532 Tuttlingen (DE)
(74) Vertreter: Witte, Weller & Partner Patentanwälte mbB

(56) Entgegenhaltungen:
- EP-A2- 2 072 001
- WO-A1-2014/127780
- US-A- 6 117 071
- US-B1- 6 319 195

## Beschreibung

Die Erfindung betrifft ein Videoendoskop, mit einem Schaft, der einen steifen proximalseitigen Abschnitt und einen in einer Ebene seitlich aus der Mittellängsachse des Schaftes auslenkbaren distalen Abschnitt aufweist, wobei am distalen Ende des auslenkbaren Abschnittes eine Bildaufnahme und eine Beleuchtung angeordnet sind und am proximalen Ende des Schaftes ein Gehäuse angeordnet ist, das ein Steuerelement zum Steuern der Auslenkung des Schaftes aufweist, wobei im Schaft außermittig zur Mittellängsachse ein stabförmiges Zugelement verläuft, das proximalseitig mit dem Steuerelement und distalseitig mit dem distalen Endbereich des auslenkbaren distalen Abschnittes des Schaftes verbunden ist, wobei ein Bewegen des Zugelementes durch das Steuerelement nach proximal ein seitliches Auslenken des auslenkbaren Abschnittes bewirkt, wobei das stabförmige Zugelement durch eine erste Öffnung am proximalen Ende des Schaftes in einen Freiraum im Innern des Gehäuses tritt, in welchem Freiraum das proximale Ende des Zugelementes mit dem Steuerelement verbunden ist.

Ein Intubationsendoskop dieser Art ist gemäß dem Oberbegriff von Anspruch 1 aus der US 6 319 195 B1 bekannt.

Aus der US 8 998 801 B2 ist ein Endoskop bekannt, das mit einem Einführabschnitt zum Einführen in einem Körper, einem Betätigungsabschnitt, der proximalseitig des Einführabschnitts angeordnet ist, einem Effektorabschnitt, der im Einführabschnitt angeordnet und über den Betätigungsabschnitt steuerbar ist, zwei Lineargliedern, die in einer Längsrichtung des Einführabschnitts bewegbar und zur Übertragung einer Eingabe zum Effektorabschnitt ausgebildet sind, und mit einem Bremselement für die zwei Linearglieder versehen ist, das mit einem Druckstück versehen ist.

Aus der DE 101 00 533 A1 ist ein Endoskop für die Notfallintubation bekannt, das mit einem Halteteil und einem wenigstens in Teilbereichen flexibel ausgebildeten Schaft versehen ist, wobei wenigstens zwei längliche biegbare Zug- und/oder Schubmittel am Schaft in axialer Richtung in unterschiedlichen Abständen vom proximalen Ende angreifen, wobei sich die Zug- und/oder Schubmittel bis an das proximale Ende erstrecken und dort in einer Befestigungseinrichtung feststellbar und lösbar gelagert sind, und wobei das Endoskop eine Endoskospitze aufweist, deren Bewegung über Steuermittel und einer im proximalen Bereich angeordneten Steuereinrichtung steuerbar ist.

Aus der WO 2014/127780 A1 ist ein Steuermechanismus für ein Endoskop bekannt, der einen Bowdenzug mit einem äußeren Führungsrohr und einem inneren Zugdraht aufweist, wobei das äußere Führungsrohr eine erste Länge aufweist und der innere Zugdraht eine zweite Länge aufweist, die größer als die erste Länge ist, wobei das äußere Führungsrohr einen ersten Führungsrohrabschnitt und einen zweiten Führungsrohrabschnitt aufweist, und wobei der erste Führungsrohrabschnitt plastische und elastische Eigenschaften aufweist, die sich von den plastischen und elastischen Eigenschaften des zweiten Führungsrohrabschnitts unterscheiden.

Aus der US 6 117 071 A ist ein Endoskop mit einem Handstück bekannt, mit einem darauf befestigtem Betätigungselement, mit einer Spitze, in der eine Bildaufnahmeeinheit angeordnet ist, mit einem Schaft zwischen dem Handstück und der Spitzeneinheit, und mit einer in dem Schaft angeordneten Betätigungseinrichtung für die Bildaufnahmeeinheit, die die Bildaufnahmeeinheit im Schaft bewegt. Zur Betätigung ist ein Kabel vorgesehen, das an seinem proximalen Ende über einen kettenartigen Koppelmechanismus mit einem Betätigungshebel verbunden ist.

Zur Intubation, insbesondere bei der Notfallintubation, ist ein Instrumentarium notwendig, mit dem Mittel zur Beatmung des Patienten möglichst schnell in die Luftröhre eingebracht werden können. Solche Notfallsituationen treten insbesondere nach Unfällen auf, bei denen die Luftröhre, somit die Atmung des Patienten stark eingeschränkt oder vollständig blockiert ist. Derartige Notfallsituationen treten insbesondere bei Arbeitsunfällen, bei Verkehrsunfällen und insbesondere bei Motorradunfällen auf. Bei einer solchen Notfallintubation muss ein Instrument bereitgestellt werden, das möglichst einfach und betriebssicher handhabbar ist.

Dazu wurden Intubationsendoskope entwickelt. Auf den distalen Endbereich eines solchen Intubationsendoskops kann ein meist aus durchsichtigen Kunststoffmaterialien hergestellter Intubationstubus aufgeschoben werden, der mit diesem Intubationsendoskop unter visueller Beobachtung in die Luftröhre eingesetzt wird. Nach Setzen des Intubationstubus wird dieser aufgebläht, so dass dieser an Ort und Stelle in der Luftröhre verbleiben kann, danach wird das Intubationsendoskop vom gesetzten Tubus abgezogen. Dieser wird dann mit einem Schlauch eines Beatmungsgerätes verbunden, über das anschließend Beatmungsluft in die Luftröhre eingebracht werden kann.

Bei solchen Intubationen entscheiden oftmals Minuten oder gar Sekunden darüber, ob der Patient so rechtzeitig wieder beatmet werden kann, das sein Überleben gesichert werden kann.

Es ist ferner ein Bestreben, den Schaft des Intubationsendoskops mit einem möglichst geringen Durchmesser zu versehen, damit ein entsprechend großer und stabiler Intubationstubus aufgesetzt und in die Luftröhre eingesetzt werden kann. Da die anatomischen Strukturen bei der Intubation bei menschlichen Patienten ähnlich sind, wird ein Endoskop eingesetzt, das einen starren geradlinigen Schaftbereich aufweist, dessen distaler Endbereich seitlich ablenkbar ist. Aufgrund der Anatomie des Menschen ist ein Endoskop mit einer distalen Auslenkung in nur eine Richtung ausreichend. Es ist ergonomisch günstiger, ein solches nur in einer Richtung auslenkbares Endoskop zu verwenden, anstatt Systeme mit Auslenkungen in zwei oder möglicherweise vier Richtungen. Das Intubationsendoskop wird zunächst in geradliniger Ausrichtung in die Mundhöhle in Richtung Kehlkopf eingeschoben und dann wird der auslenkbare Abschnitt so gekrümmt, wie es dem Übergang von der Mundhöhle über den Kehlkopf in die Luftröhre des jeweiligen Patienten entspricht.

Eine weitere Anforderung ist, dass das endoskopische Visualisierungssystem möglichst gering im Durchmesser ist, um eine ausreichende Querschnittsfläche für den Intubationstubus zur Beatmung zur Verfügung stellen zu können. Bei der eingangs genannten US 6 319 195 B1 ist ein entsprechend dünner langerstreckter Schaft ausgebildet, dessen distaler Endbereich seitlich in einer Richtung auslenkbar ist. Dazu ist im Innern gegenüber der Mittellängsachse des Schafts versetzt ein etwa stabförmiges Zugelement angeordnet, dessen distales Ende mit dem auslenkbaren Bereich verbunden ist. Das proximale Ende des Zugelementes wird durch eine Öffnung in einen inneren Freiraum eines Gehäuses geführt, das am proximalseitigen Ende des Schaftes angeordnet ist. Am Gehäuse ist ein seitlich abstehendes Steuerelement in Form eines Steuerhebels vorhanden, dessen inneres Ende in den Innenraum, sprich in den Freiraum im Gehäuse hineinragt und dort mit dem proximalen Ende des Zugelementes verbunden ist. Im geradlinig ausgestreckten Zustand des Schaftes steht der Hebel seitlich vom Gehäuse weg. Wird er an das Gehäuse herangelegt, wird das proximale Ende des Zugelementes nach proximal in den inneren Freiraum im Gehäuse hineingezogen und dabei wird dann der auslenkbare Abschnitt des Schaftes seitlich ausgelenkt.

Die Rückstellung des ausgelenkten Schaftes wieder in den geradlinigen Zustand erfolgt über ein im Innern des Schaftes angeordnetes Rückstellelement, das als ein elastisches Element ausgebildet ist, das mit dem distalen Endbereich des Zugelementes fest verbunden ist. Beim seitlichen Auslenken des Schaftes wird in diesem Rückstellelement eine Rückstellkraft aufgebaut, die nach Freigabe des Hebels für eine geradlinige Ausrichtung des Schaftes sorgt.

Je nachdem wie das Bildaufnahme- und Beleuchtungssystem ausgebildet ist, über das die Vorgänge am distalen Ende des Schaftes während der Intubation visuell erfasst werden können, müssen entsprechende Versorgungsleitungen ebenfalls durch den Schaft geführt werden, also Lichtleiter, Bildleiter oder entsprechende elektrische Versorgungsleitungen.

Da das Zugelement im praktischen Einsatz auf Zug belastet wird, also nach proximal verschoben wird, kann es relativ durchmessergering ausgebildet werden, beispielsweise als dünner Drahtkörper, über den erhebliche Zugkräfte ausgeübt werden können.

Beim Auslenken des Schaftes wird der proximale Endbereich des Zugelementes, der mit dem Steuerelement verbunden ist, über eine bestimmte Strecke in den Freiraum im Innern des Gehäuses hineinbewegt.

Im praktischen Einsatz wurde nun festgestellt, dass bei relativ durchmessergeringen Zugelementen beim geradlinigen Ausrichten des Schaftes, bei dem der im Innenraum freiliegende Bereich des Zugelementes wieder nach distal verschoben wird, Ausbauchungen oder seitliche Ausweichungen des Zugelementes erfolgen können.

Diese Ausbauchungen haben zur Folge, dass eine Bewegung des Zugelementes nach distal zum Geradestellen des Schaftes oder zur Verringerung der Krümmung des ausgelenkten Bereich zur Anpassung an die jeweilige Anatomie des Patienten durch solche Ausbauchungen oder Ausweichungen gehemmt oder blockiert ist.

Es ist daher Aufgabe der vorliegenden Erfindung hier Abhilfe zu schaffen und ein Endoskop bereitzustellen, das ergonomisch und betriebssicher und insbesondere auch als Intubationsendoskop einsetzbar ist, und bei dem die Hin- und Herbewegung des Zugelementes, insbesondere das Verschieben nach distal, betriebssicher abläuft.

Erfindungsgemäß wird die Aufgabe dadurch gelöst, dass um die Außenseite des Zugelementes ein Schubrohr gelegt ist, das sich von der proximalen Verbindung des Zugelementes mit dem Steuerelement durch die erste Öffnung bei gestrecktem Schaft in den Schaft hinein erstreckt, wobei die Länge des Schubrohres so bemessen ist, dass nach maximalem Bewegen des Zugelementes nach proximal der gesamte im Freiraum im Innern des Gehäuses liegende Abschnitt des Zugelementes vom Schubrohr belegt ist.

Das Schubrohr umgibt das Zugelement in dem gegenüber seitlichen Ausbauchungen anfälligen Abschnitt im Freiraum im Innern des Gehäuses und verhindert dadurch ein seitliches Ausweichen oder Ausbauchen des Zugelementes bei dessen Bewegung nach distal in den Schaft hinein. Es müssen keine konstruktiven Änderungen am Zugelement selbst vorgenommen werden, insbesondere muss nicht dessen Durchmesser vergrößert oder dessen Material verstärkt werden, sondern es wird in dem kritischen Bereich im Innern des Gehäuses, in dem genügend Raum zur Verfügung steht, um die Außenseite des Zugelementes das Schubrohr vorgesehen. Der Zusammenbau aus Zugelement und um dessen Außenseite gelegten Schubrohr weist dann insgesamt eine solche Steifigkeit auf, dass auch ruckartige Bewegungen des Zugelementes nach distal ohne seitliche Ausweichbewegungen oder Ausbauchungen ablaufen.

Das erhöht erheblich die Betriebssicherheit und auch die Handhabbarkeit des Intubationsendoskops, ohne dass im Innern des Schaftes, in dem wenig Bauraum zur Verfügung steht, verstärkende Maßnahmen ergriffen werden müssen.

In einer weiteren Ausgestaltung der Erfindung sind das proximale Ende des Schubrohrs und das Zugelement fest mit dem Steuerelement verbunden.

Diese Maßnahme hat den Vorteil, dass dies nicht nur technisch einfach durchzuführen ist, sondern dass durch diese feste Verbindung mit dem Steuerelement sichergestellt ist, dass keine Relativbewegungen in Längsrichtung zwischen dem Zugelement und dem angelegten Schubrohr entstehen. Auch dies trägt zur Betriebssicherheit bei.

In einer weiteren Ausgestaltung der Erfindung endet, von proximal nach distal gesehen, das distale Ende des Schubrohres bei ausgestrecktem Schaft vor dem proximalen Ende des auslenkbaren Abschnittes des Schaftes.

Diese Maßnahme hat den Vorteil, dass die konstruktiven Gegebenheiten im Bereich des auslenkbaren Abschnittes unbeeinflusst von dem Schubrohr sind. Das Schubrohr dient zwar zur Erhöhung der Steifigkeit des Zugelementes gegenüber seitlichem Ausbauchen und Krümmen, allerdings nur im Bereich des Freiraumes im Gehäuse. Im Bereich des auslenkbaren Abschnittes muss sich das Zugelement krümmen, denn dieses Zugelement bewirkt beim Bewegen nach proximal das Krümmen des auslenkbaren Abschnittes. Dadurch, dass das Schubrohr vor dem auslenkbaren Abschnitt endet, beeinflusst es nicht die Funktion, die konstruktiven Maßnahmen und die Betriebssicherheit der Auslenkung.

In einer weiteren Ausgestaltung der Erfindung ist die Länge des Schubrohres derart ausgebildet, dass nach maximalem Bewegen des Zugelementes nach proximal sich das Schubrohr nach wie vor durch die erste Öffnung hindurch in den Schaft hinein erstreckt.

Diese Maßnahme trägt zusätzlich zur Betriebssicherheit bei, und zwar im Sinne einer einwandfreien Führung des Zusammenbaus aus Zugelement und Schubrohr durch die Öffnung in den Schaft hinein.

In einer weiteren Ausgestaltung der Erfindung ist das Schubrohr in einer Führung im steifen proximalen Abschnitt des Schafts führbar.

Diese Maßnahme verstärkt noch den zuvor erwähnten Effekt, d.h. das Schubrohr samt dem darin aufgenommenen Zugelement wird im steifen proximalen Abschnitt des Schaftes betriebssicher geführt.

In einer weiteren Ausgestaltung der Erfindung erstreckt sich vom proximalen Ende der ersten Öffnung ein Führungsrohr der Führung in den Freiraum hinein, wobei der Zusammenbau aus Schubrohr und Zugelement durch das Führungsrohr hindurchgeführt ist.

Diese Maßnahme trägt nunmehr noch dahingehend weiter zur betriebssicheren Führung des Zusammenbaus aus Schubrohr und Zugelement vor, dass auch eine Führung über einen gewissen Bereich im Freiraum erfolgt, der für solche Führungsmaßnahmen zur Verfügung steht.

In einer weiteren Ausgestaltung der Erfindung ist die Länge des sich in den Freiraum hinein erstreckenden Führungsrohres so bemessen, dass bei ausgestrecktem Schaft nahezu der gesamte Abschnitt des Zusammenbaus aus Zugelement und Schubrohr zwischen der ersten Öffnung und der Verbindung mit dem Steuerelement vom Führungsrohr umgeben ist.

Diese Maßnahme stellt sicher, dass bei geradlinig ausgerichtetem Schaft, bei dem das Steuerelement maximal nach distal verschoben ist, der Bereich zwischen der Verbindung des Zusammenbaus mit dem distalen Ende des Steuerelementes und der Öffnung, über die dieser Zusammenbau in den Schaft eintritt, nahezu über seine gesamte Länge von dem Führungsrohr umgeben ist. Diese Maßnahme trägt erheblich zur Betriebssicherheit bei.

In einer weiteren Ausgestaltung der Erfindung ist das Schubrohr aus metallischem Material hergestellt.

Diese Maßnahme hat den Vorteil, dass schon mit sehr dünnen Metallrohren beispielsweise aus Edelstahl eine ausreichende Verstärkung des Zugelementes gegenüber seitlichen Ausweichbewegungen oder Ausbauchungen gegeben ist, wodurch selbige verhindert werden können.

Die Materialwahl und der Durchmesser des Zugelementes und die Wandstärke des Schubrohres sind derart aufeinander abgestimmt, dass vom Zugelement bei möglichst durchmessergeringer Bauweise die notwendigen Zugkräfte zum Auslenken des Schaftes betriebssicher ausübbar sind und durch das Schubrohr ein seitliches Ausweichen des Zugelementes im Freiraum beim linearen Ausrichten des Schaftes verhinderbar ist.

Diese Abstimmungsmaßnahme hat den Vorteil, dass man bei der Konstruktion eines solchen Intubationsendoskops den Durchmesser des Zugelementes gegebenenfalls noch weiter verringern kann, so dass extrem dünne Schäfte gebaut werden können. Wie eingangs erwähnt ist das ein Ziel, um möglichst große und stabile Intubationstuben setzen zu können. Der Durchmesser des Zugelementes im Schaft kann so gering bemessen werden, dass durch dieses gerade ausreichend Zugkräfte betriebssicher überbracht werden können, um eine Auslenkung des Schaftes zu bewirken. Der kritische freiliegende Bereich im inneren Freiraum des Schubrohres kann nunmehr dazu ausgenützt werden, um entsprechend steife oder stabile Schubrohre über den proximalen Endbereich des Zugelementes zu schieben, der beim Auslenken des Schaftes nach proximal im Gehäuse eingezogen und somit freiliegend wird. In anderen Worten ausgedrückt eröffnet die Erfindung nun die Möglichkeit, noch schlankere Schäfte mit noch schlankeren Zugelementen zu realisieren.

In einer weiteren Ausgestaltung der Erfindung erstreckt sich durch den Schaft ein Versorgungskabel, das distalseitig mit der als Einheit ausgebildeten Bildaufnahme und Beleuchtung verbunden ist, und das sich durch eine zweite Öffnung am proximalen Ende des Schaftes in den Freiraum erstreckt.

Im Rahmen der Miniaturisierung von Beleuchtung und Bildaufnahme ist es nunmehr möglich, am distalen Ende des Schaftes eine extrem durchmessergeringe Video-/LED-Einheit anzuordnen, die ein hervorragendes Bild bei ausreichender Beleuchtung zur Verfügung stellt. Das dazu notwendige Versorgungskabel muss ebenfalls durch den Schaft geführt werden und kann dann durch eine zweite Öffnung in den Freiraum des Gehäuses geführt werden, ohne dabei den Zusammenbau aus Schubrohr und Zugdraht zu beeinflussen.

In einer weiteren Ausgestaltung der Erfindung ist das Versorgungskabel als Flachbandkabel ausgebildet und ein Führungskanal im auslenkbaren Abschnitt ist im Querschnitt als Langloch ausgebildet, wobei die längere Achse der Langlochöffnung sich quer zu der Verschwenkebene des auslenkbaren Abschnittes des Schaftes erstreckt.

Diese Maßnahme hat den Vorteil, dass über ein einziges Kabel sämtliche Energie und Informationen von bzw. zu der distalseitigen Video-/LED-Einheit geführt werden können. In der Ausrichtung der Langlochöffnung wird durch das Flachbandkabel beim Auslenken eine möglichst geringe Widerstandskraft aufgebracht.

In einer weiteren Ausgestaltung der Erfindung ist im auslenkbaren Abschnitt des Schaftes eine Reihe an aneinander liegenden beweglichen Wirbelgliedern angeordnet, von denen zumindest das distalseitige mit dem distalen Ende des Zugelementes verbunden ist.

Diese an sich bekannte Maßnahme der Ausgestaltung des auslenkbaren Bereiches mit Wirbelgliedern hat den Vorteil, dass dadurch einfach und betriebssicher auslenkbare Abschnitte aufgebaut werden können, die auch eine ausreichende mechanische Stabilität aufweisen.

Diese Wirbelglieder werden üblicherweise in einer Reihe längs des Schaftes aneinander angeordnet und weisen entsprechend abgeschrägte Flächen auf. Da bei der vorliegenden Konstruktion es ausreichend ist, eine Auslenkung nur in einer Richtung vorzusehen, können auch sehr kleine durchmessergeringe Wirbelglieder eingesetzt werden, die nur an einer Seite, also in der Richtung, in der die Auslenkung erfolgen soll, etwas abgeschrägt sind. Durch Ziehen mit dem Zugelement an zumindest dem distalseitigsten Wirbelglied werden diese nach und nach über ihre schrägen Seiten aneinander gelegt und bilden dann insgesamt den ausgelenkten gekrümmten Abschnitt des Schaftes. Dies trägt ebenfalls zur Betriebssicherheit bei möglichst schlankem Schaftaufbau bei.

In einer weiteren Ausgestaltung der Erfindung ist im auslenkbaren Abschnitt des Schafts zumindest ein Rückstellelement angeordnet, in dem beim Auslenken des auslenkbaren Abschnittes eine Rückstellkraft ausbildbar ist.

Diese ebenfalls an sich bekannte Maßnahme kann im Rahmen der Erfindung dahingehend vorteilhaft eingesetzt werden, um die Rückstellung des auslenkbaren Schafts beispielsweise in Form der aneinander liegenden Wirbelglieder zu fördern.

Dies kann beispielsweise durch elastische drahtförmige Körper bewerkstelligt werden, die durch Öffnungen in den Wirbelgliedern geführt sind. So können beispielsweise entsprechende metallische Drähte oder Drähte mit Memory-Gedächtnis, wie beispielsweise Nitinoldrähte eingezogen werden, die für eine Rückstellung des auslenkbaren Bereiches sorgen. Auch das kann im Sinne der Erfindung durch betriebssichere und sehr schlankbauende Maßnahmen im auslenkbaren Bereich des Schaftes realisiert werden.

Bei Nitinol handelt es sich um eine Formgedächtnis-Legierung. Eine Nitinol-Legierung ist eine Nickel-Titan-Legierung. Andere Arten von Formgedächtnis-Legierungen sind denkbar.

Gemäß einer weiteren Ausgestaltung umfasst das Endoskop ein einziges Zugelement, das zum Auslenken des distalen Abschnitts im Schaft angeordnet ist. Insbesondere für die Notfallintubation ist es von Vorteil, das Endoskop in lediglich einer Richtung auslenkbar zu gestalten. Die Bedienung gestaltet sich einfacher. Somit können etwa Bedienfehler vermieden werden. Insbesondere in Kombination mit dem zumindest einen Rückstellelement kann mit lediglich einem einzigen Zugelement sowohl ein Auslenken des Schaftes als auch eine Rückkehr in den ausgestreckten Zustand bewirkt werden. Auch dies kann zur Bauraumreduzierung beitragen.

Gemäß einer weiteren Ausgestaltung sind das Schubrohr und das Zugelement relativ zueinander unverschiebbar an einem Zugbolzen festgelegt, der mit dem Steuerelement gekoppelt ist. Mit anderen Worten gibt es gemäß dieser Ausgestaltung keine Relativbewegung in der Längsrichtung/Längserstreckung des Zugelements zwischen dem Zugelement und dem Schubrohr. Die relative Fixierung zwischen dem Schubrohr und dem Zugelement kann durch die gemeinsame Befestigung am Zugbolzen bewerkstelligt werden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels in Form eines Videointubationsendoskopes in Zusammenhang mit den beiliegenden Zeichnungen näher beschrieben und erläutert. Es zeigen:
- Fig. 1: schematisch stark vereinfacht und teilweise aufgebrochen eine Seitenansicht eines Endoskops in ausgestrecktem Schaftzustand mit aufgeschobenem Intubationstubus;
- Fig. 2: eine entsprechende Seitenansicht, bei der der auslenkbare Bereich des Schaftes in einer Richtung ausgelenkt ist und der Intubationstubus zum Verankern in der Luftröhre aufgebläht ist;
- Fig. 3: einen Längsschnitt lediglich des Schaftes des Endoskops von Fig. 1 und 2 im ausgestreckten Zustand;
- Fig. 4: eine vergrößerte Darstellung des in Fig. 3 mit dem Kreis IV umgrenzten Bereiches;
- Fig. 5: eine vergrößerte Darstellung des in Fig. 3 mit dem Kreis V umgrenzten Bereiches;
- Fig. 6: eine vergrößerte Darstellung des in Fig. 3 mit dem Kreis VI umgrenzten Bereiches;
- Fig. 7: eine der Fig. 3 entsprechende Schnittdarstellung des Schaftes im seitlich ausgelenkten Zustand;
- Fig. 8: eine vergrößerte Darstellung des in Fig. 7 mit dem Kreis VIII umgrenzten Bereiches;
- Fig. 9: eine vergrößerte Darstellung des in Fig. 7 mit dem Kreis IX umgrenzten Bereiches;
- Fig. 10: einen Querschnitt durch ein Wirbelglied längs der Linie X-X in Fig. 3;
- Fig. 11: einen Schnitt längs der Linie XI-XI in Fig. 10; und
- Fig. 12: einen Schnitt längs der Linie XII-XII in Fig. 10.

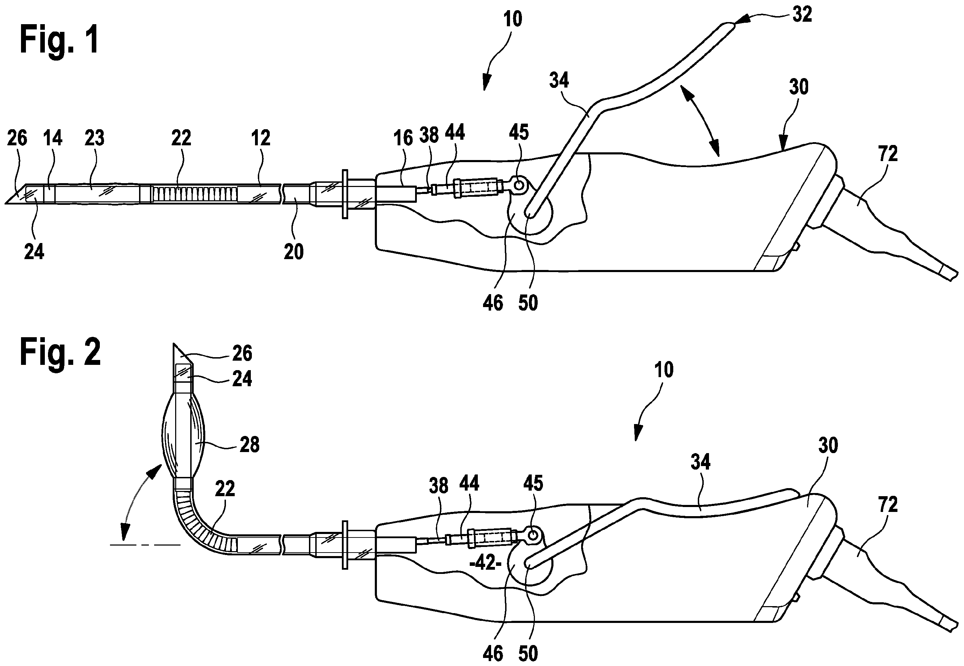

Ein in den Fig. 1 bis 10 dargestelltes Endoskop ist ein Videointubationsendoskop, das in seiner Gesamtheit mit der Bezugsziffer 10 bezeichnet.

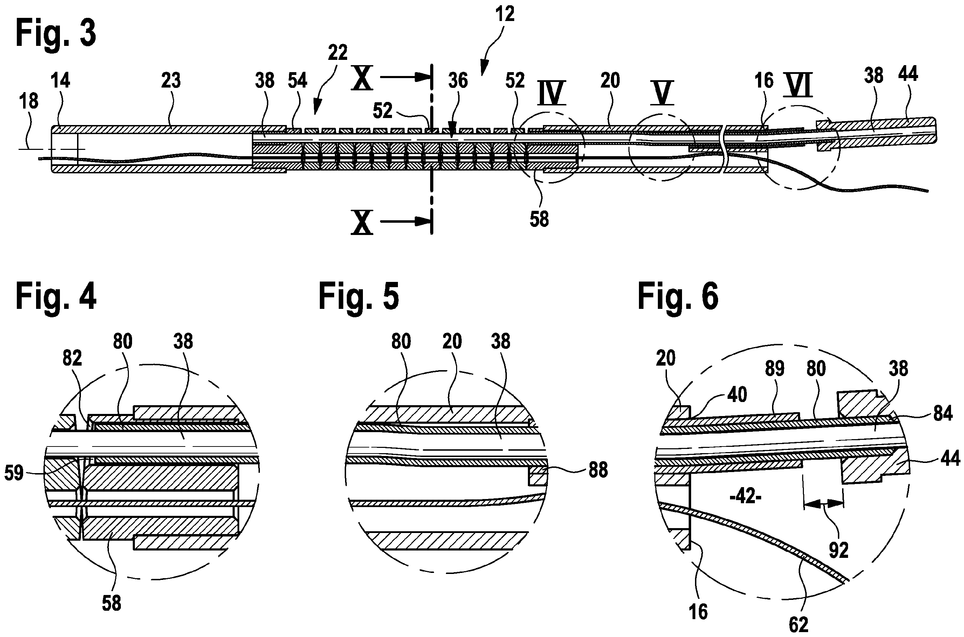

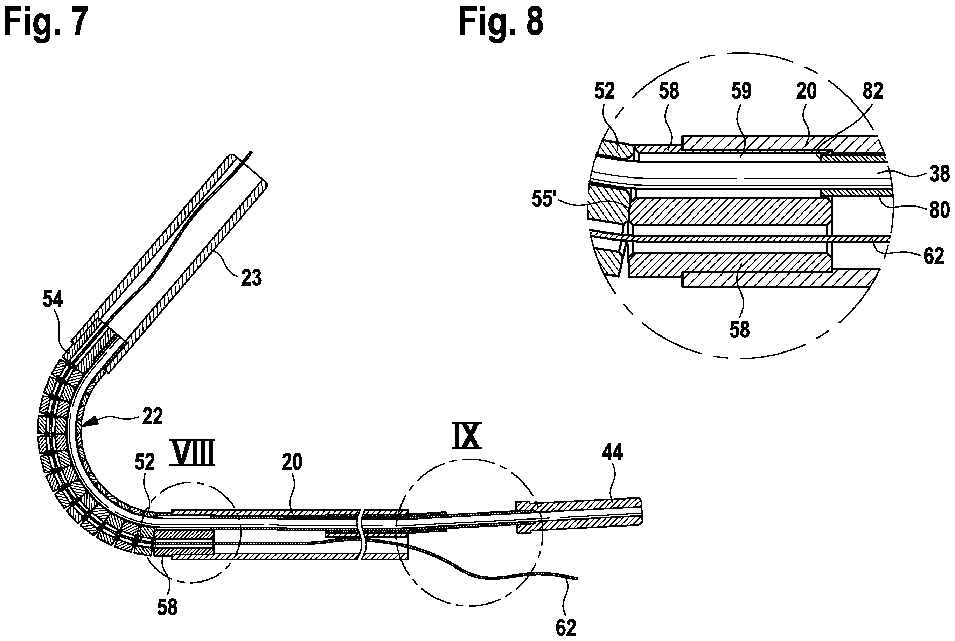

Wie insbesondere aus Fig. 1 und 2 ersichtlich, weist das Videointubationsendoskop 10 einen Schaft 12 auf, der in den Fig. 3 und 7 isoliert dargestellt ist.

Der Schaft 12 weist ein distales Ende 14 und ein proximales Ende 16 auf. Das proximale Ende 14 ist das einem Bediener zugewandte bzw. von einem Patienten abgewandte Ende. Das distale Ende 16 ist das vom Bediener abgewandte bzw. dem Patienten zugewandte Ende. Der Schaft 12 weist eine Mittellängsachse 18 auf und kann zwischen einem geradlinig ausgestreckten Zustand, wie er in den Fig. 1 und 3 dargestellt ist und einem in eine Richtung seitlich ausgelenkten Zustand, wie er in den Fig. 2 und 7 dargestellt ist, bewegt werden.

Der Schaft 12 weist einen steifen proximalen rohrförmigen Abschnitt 20, einen distal daran anschließenden auslenkbaren distalen Abschnitt 22 und ein steifes hohlrohrförmiges Ende 23 auf.

Wie insbesondere aus den Fig. 1 und 2 zu erkennen, ist an dem distalen Ende 14 des Schaftes 12 eine Bildaufnahme/Beleuchtungs-Einheit 24 montiert.

Diese Einheit 24 ist als ein kompaktes Bauelement ausgebildet, das einen Bildsensor als Bildaufnahme beinhaltet, sowie zumindest eine LED als Beleuchtung. Die energetische Versorgung sowie die Übermittlung der Bildinformation erfolgt über ein Versorgungskabel 62, das als Breitbandkabel ausgeführt ist, und das sich vom distalen Ende des Schaftes durch diesen hindurch zu einem Gehäuse 30 erstreckt, das am proximalen Ende 16 des Schaftes 12 angeordnet ist. Das Versorgungskabel 62 erstreckt sich durch den Innenraum des Gehäuses 30 und wird an dessen proximalem Ende über eine Kabeltülle 72 zu entsprechenden Versorgungsquellen bzw. einem Monitor geführt, um das Bild zu visualisieren.

Somit handelt es sich um ein Videointubationsendoskop.

In den Fig. 1 und 2 ist dargestellt, dass auf das steife Ende 23 des Schaftes 12 ein flexibler endotrachealer Tubus 26 aufgeschoben ist, der mit dem Videointubationsendoskop 10 bei einer Intubation in eine Luftröhre eines Patienten gesetzt werden soll.

Wie im Bereich der Intubation üblich, wird nach Einbringen des Zusammenbaus aus Videointubationsendoskop 10 und endotrachealem Tubus 26 an die geeignete Stelle in der Luftröhre der aufblähbare Bereich 28 des Tubus 26 aufgebläht, so dass dieser dann in der Luftröhre vor Ort verankert ist. Danach kann das Videointubationsendoskop 10 vom gesetzten Tubus 26 abgezogen werden. Zum Aufblähen ist üblicherweise eine Spritze vorgesehen, die über eine dünne Leitung mit dem Tubus 26 verbunden ist, um den aufblähbaren Bereich 28 mit Luft aufzublähen. Der Übersichtlichkeit halber ist das hier nicht dargestellt, da der Tubus keinen Teil der Erfindung darstellt.

Wie insbesondere aus den Fig. 1 und 2 ersichtlich, ist das Gehäuse 30 mit einem Steuerelement 32 in Form eines davon seitlich abstehenden Hebels 34 versehen, der beispielsweise als das Gehäuse 30 U-förmig umgreifender Bügel ausgebildet sein kann. Das innere gehäuseseitige Ende 50 des Steuerelementes 32 reicht in das Innere des Gehäuses 30 hinein, wie das durch die gebrochene Darstellung von Fig. 1 und 2 ersichtlich ist.

Dort ist das Steuerelement 32 mit dem proximalen Ende eines stabförmigen Zugelementes 36 in Form eines Zugdrahtes 38 verbunden, wie das nachfolgend noch anhand der weiteren Zeichnungen näher beschrieben und erläutert wird. Das Zugelement 36 erstreckt sich durch den Schaft 12 hindurch und dient dazu, den auslenkbaren Abschnitt 22 des Schaftes 12 aus der in Fig. 1 geradlinig ausgestreckten Orientierung in die in Fig. 2 dargestellte seitlich ausgelenkte gekrümmte Orientierung zu bewegen.

Wie insbesondere aus Fig. 1 und 2 zu erkennen, ragt dazu der proximale Endbereich des Zugelementes 36 über das proximale Ende 16 des Schaftes hinaus und in einen inneren Freiraum 42 im Innern des Gehäuses 30 hinein.

Dort ist das Zugelement 36 mit einem Zugbolzen 44 verbunden, der schwenkbar über einen Achsbolzen 45 an einer Exzenterscheibe 46 angeordnet ist.

Im Abstand zu dem Achsbolzen 45 ist auch das innere Ende 50 des zuvor erwähnten Steuerelements 32 an der Exzenterscheibe 46 befestigt.

Wird daher der Hebel 34 aus der in Fig. 1 ersichtlichen seitlich abstehenden Position vom Gehäuse 30 auf dieses zu bewegt, wie das dem Übergang von Fig. 1 zu Fig. 2 entspricht, wird durch die Exzenterscheibe 46 der Zugbolzen 44 nach proximal bewegt und bewegt damit auch das Zugelement 36 nach proximal in den Freiraum 42 im Gehäuse 30 hinein, wie das ebenfalls aus dem Übergang von Fig. 1 zu Fig. 2 ersichtlich ist.

Dabei wird dann der Schaft 12 entsprechend ausgelenkt.

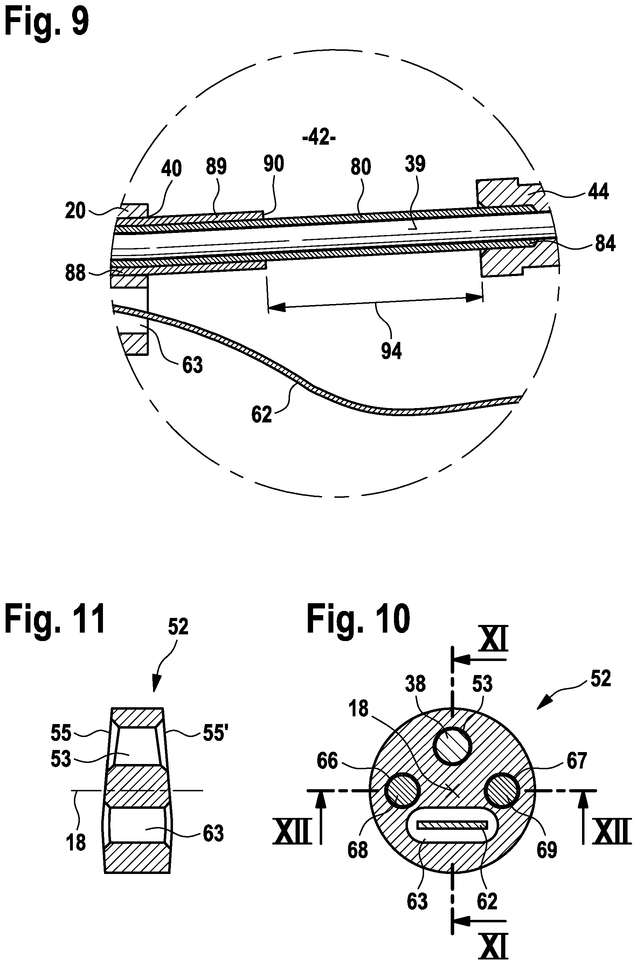

Die nähere Ausgestaltung des Schaftes 12 und dessen weitere konstruktiven Elemente sollen in Zusammenhang mit den Fig. 3 bis 11 erläutert werden. Der auslenkbare distale Abschnitt 22 ist im dargestellten Ausführungsbeispiel aus zwölf längs der Mittellängsachse 18 aneinander gereihten Wirbelgliedern 52 aufgebaut, deren näherer konstruktiver Aufbau in Fig. 10 und 11 ersichtlich ist. Diese zwölf Wirbelglieder 52 sind zwischen einem distalen Endstück 54 und einem proximalen Endstück 58 gehalten.

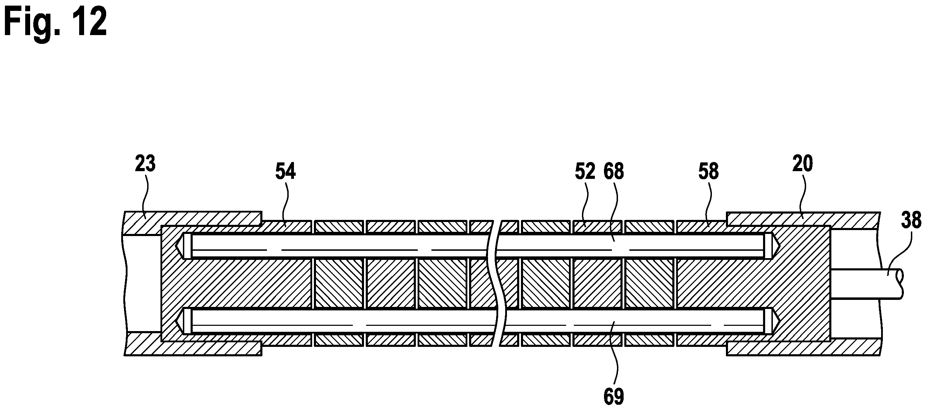

Das distale Endstück 54 ist dabei in das proximale Ende des rohrförmigen Endstückes 23 eingeschoben. Dementsprechend ist das etwa gleich ausgebildete proximale Endstück 58 spiegelbildlich angeordnet und in das distale Ende des steifen proximalen Abschnittes 20 des Schaftes eingeschoben, wie das insbesondere aus den Fig. 3 und 12 ersichtlich ist. Durch die Wirbelglieder 52 und durch die Endstücke 54 und 58 hindurch erstreckt sich eine seitlich versetzt zur Mittellängsachse 18 erstreckende durchgehende Bohrung 53. In diesen durchgehenden Bohrungen ist das Zugelement 36 in Form eines dünnen metallischen Drahtes 38 aufgenommen. Wie insbesondere aus der Darstellung von Fig. 3 zu entnehmen, ist das distale Ende des Drahtes 38 im distalen Endstück 54 über eine Klemmhülse ortsfest fixiert. Am proximalen Ende ist der Draht 38 aus dem proximalen Endstück 48 weiter durch den steifen proximalen Abschnitt 20 des Schaftes 12 geführt und tritt über eine erste endseitige Öffnung 40 aus dem Schaft 12 in den Freiraum 42 ins Gehäuse 30 ein. Dort ist das distale Ende des Drahtes 38 mit dem zuvor erwähnten Zugbolzen 44 verbunden.

Wie insbesondere aus der Schnittdarstellung von Fig. 11 zu erkennen, weisen die Wirbelglieder 52 in dem Bereich, in dem die Bohrung 53 mit dem durchgeführten Draht 38 vorhanden ist, gegenüberliegende etwas nach innen gerichtete Abschrägungen 55, 55' auf.

Wird daher an dem Draht 38 mittels des Zugbolzens 44 nach proximal gezogen, werden die einzelnen Wirbelglieder 52 so weit gegeneinander verkippt, bis diese sich jeweils über ihre Abschrägungen 55, 55' aneinander legen. Dies ist insbesondere aus dem Übergang von Fig. 3 zu Fig. 7 zu erkennen. Dadurch entsteht dann der ausgelenkte Abschnitt 22.

Im endfertigen Bauzustand ist dieser Bereich durch eine hier nicht dargestellte äußere flexible Schutzhülle abgedeckt.

Aus den Fig. 10 und 11 ist ferner zu erkennen, dass bezüglich der Mittellängsachse 18 gegenüberliegend zur Bohrung 53 im Abstand zu der Mittellängsachse 18 eine weitere Bohrung 63 vorgesehen ist, die im Querschnittsprofil als Langlochöffnung ausgebildet ist. Diese dient dazu, um das Versorgungskabel 62 in Form eines Flachbandkabels durch den Schaft hindurch zu führen. Dabei ist die Lage des Langloches der Langlochöffnung 63 so gewählt, dass deren längere Achse sich quer zu der Ebene erstreckt, in der der Schaft auslenkbar ist. Dadurch entsteht im auslenkbaren Abschnitt 22 ein durchgehender Führungskanal für das Flachbandkabel.

In der Darstellung von Fig. 10 entspricht die gestrichelte Linie XI-XI dieser Auslenkebene. In dieser Richtung kann das Flachbandkabel 62 einfach und ohne großen Kraftaufwand gebogen werden.

Wie aus Fig. 10, 11 und Fig. 12 zu entnehmen, sind in den Wirbelgliedern 52 noch zwei weitere Bohrungen 66 und 67 vorhanden, die in den Endstücken 54 und 58 als Sacklochbohrungen enden.

Diese Bohrungen 66 und 67 dienen zur Aufnahme von draht- bzw. stabförmigen Rückstellelementen 68 und 69, die im dargestellten Ausführungsbeispiel aus Nitinoldrähten bestehen.

Diese Rückstellelemente 68, 69werden beim Übergang von der Gestaltung von Fig. 1 zur Fig. 2 gebogen, und dabei wird eine Rückstellkraft aufgebaut. Wird somit der Hebel 34 in der in Fig. 2 dargestellten Stellung freigegeben, sorgt die Rückstellkraft der Rückstellelemente 68, 69 dazu, dass sich der Schaft wieder in die in Fig. 1 dargestellte linear ausgerichtete Position bewegt.

Aus den Schnittdarstellungen von Fig. 3 bis 6 ist die nähere Ausgestaltung des proximalen Endbereiches des Schaftes 12 ersichtlich.

Aus Fig. 3 und 6 ist ersichtlich, dass am proximalen Ende 16 des Schaftes 12 eine erste Öffnung 40 vorgesehen ist, in der eine Führung 88 in Form eines Führungsrohres 89 eingesetzt ist. Die Führung 88 erstreckt sich distalseitig über einen gewissen Längenabschnitt in das Innere des steifen proximalen Abschnittes 20 hinein, wie das insbesondere aus Fig. 3 und 5 ersichtlich ist.

Aus Fig. 6 ist ersichtlich, dass sich das Führungsrohr 89 auch proximalseitig vom proximalen Ende 16 des Schaftes 12 weg und in den Freiraum 42 hinein erstreckt. Die Länge der Erstreckung in den Freiraum 42 ist so gewählt, dass bei der in Fig. 3 und Fig. 6 dargestellten gestreckten Stellung des Schaftes 12 nur ein relativ geringer Abstand 92 zwischen dem proximalen Ende des Führungsrohres 89 und dem diesen gegenstehenden distalen Ende des Zugbolzens 44 besteht.

Aus den Fig. 3 bis 9 ist ferner zu erkennen, dass um die Außenseite 39 des Drahtes 38 ein Schubrohr 80 geschoben bzw. gelegt ist. Das heißt die Innenseite des Schubrohres 80 liegt an der Außenseite 39 des Drahtes an. Das Schubrohr 80 ist aus einer metallischen Rohrhülse aus Edelstahl hergestellt.

Wie insbesondere aus der Darstellung von Fig. 6 zu erkennen, ist das proximale Ende 84 des Schubrohres 80 in einer entsprechenden Bohrung im Zugbolzen 44 ortsfest montiert. In anderen Worten ausgedrückt ist somit in diesem Bereich sowohl das Schubrohr 80 als auch der Draht 38 fest und relativ nicht zueinander verschiebbar am Zugbolzen 44 montiert.

Das Schubrohr 80 erstreckt sich proximalseitig durch das Führungsrohr 89 und die Führung 88 so weit in das Innere des steifen proximalen Abschnittes 20 des Schaftes 12 hinein, dass es in das proximale Endstück 58 hineinreicht und darin geführt ist. Dies ist insbesondere aus Fig. 4 zu erkennen, d.h. in dem linear ausgestreckten Zustand des Schaftes endet das distale Ende 82 des Schubrohres 80 etwa auf Höhe des distalen Endes des proximalen Endstückes 58. Distalseitig dieser Stelle verläuft dann nur noch das Zugelement 36 durch die Wirbelglieder 52 und nicht das Schubrohr 80.

Wird nunmehr der Schaft 12 gekrümmt, wie das in Fig. 2 und in Fig. 7 dargestellt ist, bewegt sich der Zugbolzen 44 nach proximal und der Zusammenbau aus Schubrohr 80 und Draht 38 wird nach proximal bewegt. In Fig. 9 ist dieser maximale Hub dargestellt. Daraus ist ersichtlich, dass nunmehr der Abstand 94 zwischen dem proximalen Ende 90 des Führungsrohres 89 und dem diesen gegenüberliegenden stirnseitigen Ende des Zugbolzens 44 wesentlich größer ist als der in Fig. 6 dargestellte Abstand 92.

In diesem Bereich würde der Draht 38 frei liegen, wenn er nicht erfindungsgemäß durch das Schubrohr 80 geschützt, sprich verstärkt wäre. Durch das Schubrohr 80, das sich insbesondere in diesem kritischen Abstandsbereich 94 erstreckt, wird sichergestellt, dass bei der linearen Ausstreckbewegung des Schaftes 12, wenn sich also der Zugbolzen 54 von der in Fig. 9 dargestellten Stellung in die Fig. 6 dargestellte Stellung bewegt, keine Ausbauchungen oder seitliches Ausweichen des Drahtes 38 in diesem Bereich erfolgen. Aus Fig. 8 ist zu entnehmen, dass in dem in Fig. 9 dargestellten Zustand das distale Ende 82 des Schubrohres 80 sich in der Führung 88 im Innern des Schaftes 12 ebenfalls nach proximal bewegt hat, aber nach wie vor in dieser Führung 88 und im Endstück 58 aufgenommen ist.

Dadurch können die Verschiebebewegungen des Zugelementes 36 beim Krümmen des Schaftes und insbesondere beim anschließenden linearen Ausrichten sicher, insbesondere betriebssicher geführt und durchgeführt werden.

## Patentansprüche

1. Videoendoskop, mit einem Schaft (12), der einen steifen proximalseitigen Abschnitt (20) und einen in einer Ebene seitlich aus der Mittellängsachse (18) des Schaftes (12) auslenkbaren distalen Abschnitt (22) aufweist, wobei am distalen Ende (14) des auslenkbaren Abschnittes (22) eine Bildaufnahme und eine Beleuchtung (24) angeordnet sind, und am proximalen Ende (16) des Schaftes (12) ein Gehäuse (30) angeordnet ist, das ein Steuerelement (32) zum Steuern der Auslenkung des Schaftes (12) aufweist, wobei im Schaft (12) außermittig zur Mittellängsachse (18) ein stabförmiges Zugelement (36) verläuft, das proximalseitig mit dem Steuerelement (32) und distalseitig mit dem distalen Endbereich des auslenkbaren distalen Abschnittes (22) des Schaftes (12) verbunden ist, wobei ein Bewegen des Zugelementes (36) durch das Steuerelement (32) nach proximal ein seitliches Auslenken des auslenkbaren Abschnittes bewirkt, wobei das stabförmige Zugelement (36) durch eine erste Öffnung (40) am proximalen Ende (16) des Schaftes (12) in einen Freiraum (42) im Innern des Gehäuses (30) tritt, in welchem Freiraum (42) das proximale Ende des Zugelementes (36) mit dem Steuerelement (32) verbunden ist, **dadurch gekennzeichnet, dass** um die Außenseite (39) des Zugelementes (36) ein Schubrohr (80) gelegt ist, das sich von der proximalen Verbindung des Zugelementes (36) mit dem Steuerelement (32) durch die erste Öffnung (40) bei gestrecktem Schaft (12) in den Schaft (12) hinein erstreckt, wobei die Länge des Schubrohres (80) so bemessen ist, dass nach maximalem Bewegen des Zugelementes (36) nach proximal der gesamte im Freiraum (42) im Innern des Gehäuses (30) liegende Abschnitt des Zugelementes (36) vom Schubrohr (80) belegt ist.

2. Videoendoskop nach Anspruch 1, **dadurch gekennzeichnet, dass** das proximale Ende (84) des Schubrohres (80) und das Zugelement (36) fest mit dem Steuerelement (32) verbunden sind.

3. Videoendoskop nach Anspruch 1 oder 2, **dadurch gekennzeichnet, dass**, von proximal nach distal gesehen, das distale Ende (82) des Schubrohres (80) bei ausgestrecktem Schaft (12) vor dem proximalen Ende des auslenkbaren Abschnittes (22) des Schaftes (12) endet.

4. Videoendoskop nach einem der Ansprüche 1 bis 3, **dadurch gekennzeichnet, dass** sich das Schubrohr (80) nach maximalem Bewegen des Zugelementes (36) nach proximal nach wie vor durch die erste Öffnung (40) hindurch in den Schaft (12) hinein erstreckt.

5. Videoendoskop nach einem der Ansprüche 1 bis 4, **dadurch gekennzeichnet, dass** das Schubrohr (80) in einer Führung (88) im steifen proximalen Abschnitt (20) des Schaftes (12) geführt ist.

6. Videoendoskop nach Anspruch 5, **dadurch gekennzeichnet, dass** sich vom proximalen Ende der ersten Öffnung (40) ein Führungsrohr (89) der Führung (88) in den Freiraum (42) hinein erstreckt, wobei der Zusammenbau aus Schubrohr (80) und Zugelement (36) durch das Führungsrohr (89) hindurchgeführt ist.

7. Videoendoskop nach Anspruch 6, **dadurch gekennzeichnet, dass** die Länge des sich in den Freiraum (42) hinein erstreckenden Führungsrohres (89) so bemessen ist, dass bei ausgestrecktem Schaft (12) nahezu der gesamte Abschnitt des Zusammenbaus aus Zugelement (36) und Schubrohr (80) zwischen der ersten Öffnung (40) und der Verbindung mit dem Steuerelement (32) vom Führungsrohr (89) umgeben ist.

8. Videoendoskop nach einem der Ansprüche 1 bis 7, **dadurch gekennzeichnet, dass** das Schubrohr (80) aus metallischem Material hergestellt ist.

9. Videoendoskop nach einem der Ansprüche 1 bis 8, **dadurch gekennzeichnet, dass** sich durch den Schaft (12) ein Versorgungskabel (62) erstreckt, das distalseitig mit der als Einheit (24) ausgebildeten Bildaufnahme und Beleuchtung verbunden ist und sich durch eine zweite Öffnung (63) am proximale Ende des Schaftes (12) in den Freiraum (42) hinein erstreckt.

10. Videoendoskop nach Anspruch 9, **dadurch gekennzeichnet, dass** das Versorgungskabel (62) ein Flachbandkabel ist, und dass ein Führungskanal für das Flachbandkabel im auslenkbaren Abschnitt (22) im Querschnitt als Langlochöffnung (63) ausgebildet ist, deren längere Achse sich quer zu der Verschwenkebene des auslenkbaren Abschnittes (22) des Schaftes (12) erstreckt.

11. Videoendoskop nach einem der Ansprüche 1 bis 10, **dadurch gekennzeichnet, dass** im auslenkbaren Abschnitt (22) des Schaftes (12) eine Reihe von aneinander liegenden beweglichen Wirbelgliedern (52) angeordnet ist, von denen zumindest das distalseitige mit dem distalen Ende des Zugelementes (36) verbunden ist.

12. Videoendoskop nach einem der Ansprüche 1 bis 11, **dadurch gekennzeichnet, dass** im auslenkbaren Abschnitt (22) des Schaftes (12) zumindest ein Rückstellelement (68, 69) angeordnet ist, durch welches beim Auslenken des auslenkbaren Abschnittes (22) eine auf den Schaft (12) einwirkende Rückstellkraft ausbildbar ist.

13. Videoendoskop nach einem der Ansprüche 1 bis 12, **dadurch gekennzeichnet, dass** im Schaft (12) ein einziges Zugelement (36) zum Auslenken des distalen Abschnitts (22) angeordnet ist.

14. Videoendoskop nach einem der Ansprüche 1 bis 13, **dadurch gekennzeichnet, dass** das Schubrohr (80) und das Zugelement (36) relativ zueinander unverschiebbar an einem Zugbolzen (44) festgelegt sind, der mit dem Steuerelement (32) gekoppelt ist.

15. Videoendoskop nach einem der Ansprüche 1 bis 14, **dadurch gekennzeichnet, dass** es als Videointubationsendoskop (10) ausgebildet ist.

## Claims

1. A video endoscope, comprising a shaft (12) comprising a rigid, proximal-side section (20) and a deflectable distal section (22) that is laterally deflectable from a central longitudinal axis (18) of the shaft (12), wherein an imager and an illuminator (24) are arranged at the distal end (14) of the deflectable section (22), and wherein a housing (30) is arranged at a proximal end (16) of the shaft (12), the housing (30) comprising a control element (32) for controlling the deflection of the shaft (12), wherein a rod-like pull element (36) extends in the shaft (12) in an off-center fashion with respect to the central longitudinal axis (18), wherein the pull element (36) is, at a proximal side thereof, coupled with the control element (32) and, at a distal side thereof, coupled with the distal end region of the deflectable distal section (22) of the shaft (12), wherein a movement of the pull element (36) in the proximal direction by the control element (32) effects a lateral deflection of the deflectable section, wherein the rod-like pull element (36) extends into a clearance space (42) in the interior of the housing (30) through a first opening (40) at the proximal end (16) of the shaft (12), wherein the proximal end of the pull element (36) is coupled with the control element (32) in the clearance space (42), **characterized in that** a push tube (80) encases the exterior side (39) of the pull element (36), wherein the push tube (80) extends from the proximal connection of the pull element (36) with the control element (32) through the first opening (40) into the shaft (12), when the shaft (12) is straightened, wherein the length of the push tube (80) is defined in such a way that that the entire section of the pull element (36) that is arranged in the clearance space (42) of the interior of the housing (30) is covered by the push tube (80) when the pull element (36) is maximally moved in the proximal direction.

2. The video endoscope according to claim 1, **characterized in that** the proximal end (84) of the push tube (80) and the pull element (36) are fixedly coupled with the control element (32).

3. The video endoscope according to claim 1 or 2, **characterized in that**, seen from proximal to distal, the distal end (82) of the push tube (80) ends before the proximal end of the deflectable section (22) of the shaft (12), when the shaft (12) is straightened.

4. The video endoscope according to any one of claims 1 to 3, **characterized in that** the push tube (80) still extends through the first opening (40) into the shaft (12) when the pull element (36) is maximally moved in the proximal direction.

5. The video endoscope according to any one of the claims 1 to 4, **characterized in that** the push tube (80) is guided in a guide (88) in the rigid proximal section (20) of the shaft (12).

6. The video endoscope according to claim 5, **characterized in that** a guide tube (89) of the guide (88) extends from the proximal end of the first opening (40) into the clearance space (42), wherein the assembly of push tube (80) and pull element (36) passes through the guide tube (89).

7. The video endoscope according to claim 6, **characterized in that** the length of the guide tube (89) that extends into the clearance space (42) is defined in such a way that nearly the entire section of the assembly of pull element (36) and push tube (80) between the first opening (40) and the connection with the control element (32) is encompassed by the guide tube (89), when the shaft (12) is straightened.

8. The video endoscope according to any one of the claims 1 to 7, **characterized in that** the push tube (80) is made from metal material.

9. The video endoscope according to any one of the claims 1 to 8, **characterized in that** a supply cable (62) extends through the shaft (12), wherein the supply cable (62) is, at a distal side thereof, coupled with the imager and the illuminator that are formed as a unit (24), and wherein the supply cable (62) extends through a second opening (63) at the proximal end of the shaft (12) into the clearance space (42).

10. The video endoscope according to claim 9, **characterized in that** the supply cable (62) is a flat ribbon cable, and that a guide channel for the flat ribbon cable is provided in the deflectable section (22), the guide channel forming, in cross-section, an elongated hole opening (63) the longer axis of which extends transversely to a swivel plane of the deflectable section (22) of the shaft (12).

11. The video endoscope according to any one of the claims 1 to 10, **characterized in that** a series of adjacent moveable vertebral members (52) is arranged in the deflectable section (22) of the shaft (12), wherein at least the distal-side member of the series is coupled with the distal end of the pull element (36).

12. The video endoscope according to any one of the claims 1 to 11, **characterized in that** at least one rebound element (68, 69) is arranged in the deflectable section (22) of the shaft (12), wherein the at least one rebound element (68, 69) is arranged to generate a reset force acting on the shaft (12), when the deflectable section (22) is deflected.

13. The video endoscope according to any one of the claims 1 to 12, **characterized in that** a single pull element (36) for deflecting the distal section (22) is arranged in the shaft (12).

14. The video endoscope according to any one of the claims 1 to 13, **characterized in that** the push tube (80) and the pull element (36) are fixedly attached, in a manner non-displaceable with respect to one another, to a pull bolt (44) that is operatively coupled with the control element (32).

15. The video endoscope according to any one of the claims 1 to 14, **characterized in that** the video endoscope is arranged as a video intubation endoscope (10).

## Revendications

1. Endoscope vidéo comprenant une tige (12) qui comporte une partie proximale rigide (20) et une partie distale (22) qui peut être déviée de l'axe longitudinal central (18) de la tige (12) latéralement dans un plan, un capteur d'image et un moyen d'éclairage (24) étant disposés à l'extrémité distale (14) de la partie (22) pouvant être déviée et un boîtier (30) étant disposé à l'extrémité proximale (16) de la tige (12), lequel boîtier comporte un élément de commande (32) destiné à commander la déviation de la tige (12), un élément de traction (36) en forme de barre s'étendant dans la tige (12) de manière excentrée par rapport à l'axe longitudinal central (18), lequel élément de traction étant relié du côté proximal à l'élément de commande (32) et du côté distal à la région d'extrémité distale de la partie distale (22), pouvant être déviée, de la tige (12), le déplacement de l'élément de traction (36) par l'élément de commande (32) vers le côté proximal provoquant une déviation latérale de la partie pouvant être déviée, l'élément de traction (36) en forme de tige pénétrant dans un espace libre (42), situé à l'intérieur du boîtier (30), en passant par une première ouverture (40) ménagée à l'extrémité proximale (16) de la tige (12), espace libre (42) dans lequel l'extrémité proximale de l'élément de traction (36) est reliée à l'élément de commande (32), **caractérisé en ce qu'**un tube de poussée (80) est placé autour du côté extérieur (39) de l'élément de traction (36), lequel tube de poussée s'étend depuis la liaison proximale de l'élément de traction (36) à l'élément de commande (32) jusque dans la tige (12) en passant la première ouverture (40) lorsque la tige (12) est étendue, la longueur du tube de poussée (80) étant dimensionnée de manière à ce que, après un déplacement maximal de l'élément de traction (36) en direction du côté proximal, toute la partie de l'élément de traction (36) qui se trouve dans l'espace libre (42) situé à l'intérieur du boîtier (30) soit occupée par le tube de poussée (80).

2. Endoscope vidéo selon la revendication 1, **caractérisé en ce que** l'extrémité proximale (84) du tube de poussée (80) et l'élément de traction (36) sont reliés de manière fixe à l'élément de commande (32).

3. Endoscope vidéo selon la revendication 1 ou 2, **caractérisé en ce que**, lorsque l'on regarde depuis le côté proximal en direction du côté distal, l'extrémité distale (82) du tube de poussée (80) se termine en avant de l'extrémité proximale de la partie (22), pouvant être déviée, de la tige (12) lorsque la tige (12) est étendue.

4. Endoscope vidéo selon l'une des revendications 1 à 3, **caractérisé en ce que**, après déplacement maximal de l'élément de traction (36), le tube de poussée (80) s'étend en direction du côté proximal jusque dans la tige (12) comme précédemment en passant par la première ouverture (40).

5. Endoscope vidéo selon l'une des revendications 1 à 4, **caractérisé en ce que** le tube de traction (80) est guidé dans un guide (88) situé dans la partie proximale rigide (20) de la tige (12).

6. Endoscope vidéo selon la revendication 5, **caractérisé en ce qu'**un tube de guidage (89) du guide (88) s'étend depuis l'extrémité proximale de la première ouverture (40) jusque dans l'espace libre (42), l'ensemble formé du tube de poussée (80) et de l'élément de traction (36) passant à travers le tube de guidage (89).

7. Endoscope vidéo selon la revendication 6, **caractérisé en ce que** la longueur du tube de guidage (89) s'étendant jusque dans l'espace libre (42) est dimensionnée de manière à ce que, lorsque la tige (12) est étendue, la quasi-totalité de la partie de l'ensemble, formé de l'élément de traction (36) et du tube de poussée (80), qui est située entre la première ouverture (40) et la liaison avec l'élément de commande (32), soit entourée par le tube de guidage (89).

8. Endoscope vidéo selon l'une des revendications 1 à 7, **caractérisé en ce que** le tube de poussée (80) est fabriqué à partir d'un matériau métallique.

9. Endoscope vidéo selon l'une des revendications 1 à 8, **caractérisé en ce qu'**un câble d'alimentation (62) s'étend à travers la tige (12), lequel câble d'alimentation est relié du côté distal à l'unité (24) formée du capteur d'image et du moyen d'éclairage et s'étend jusque dans l'espace libre (42) en passant par une deuxième ouverture (63) ménagée à l'extrémité proximale de la tige (12).

10. Endoscope vidéo selon la revendication 9, **caractérisé en ce que** le câble d'alimentation (62) est un câble en nappe et **en ce qu'**un canal de guidage destiné au câble en nappe est conçu dans la partie (22), pouvant être déviée, en coupe transversale sous la forme d'une ouverture oblongue (63) dont l'axe le plus long s'étend transversalement au plan de pivotement de la partie (22), pouvant être déviée, de la tige (12).

11. Endoscope vidéo selon l'une des revendications 1 à 10, **caractérisé en ce qu'**un certain nombre d'éléments à turbulence mobiles contigus (52), dont au moins celui situé du côté distal est relié à l'extrémité distale de l'élément de traction (36), est disposé dans la partie (22), pouvant être déviée, de la tige (12).

12. Endoscope vidéo selon l'une des revendications 1 à 11, **caractérisé en ce qu'**au moins un élément de rappel (68, 69), permettant de générer une force de rappel, agissant sur la tige (12), lors de la déviation de la partie (22) pouvant être déviée, est disposé dans la partie (22), pouvant être déviée, de la tige (12).

13. Endoscope vidéo selon l'une des revendications 1 à 12, **caractérisé en ce qu'**un seul élément de traction (36) destiné à dévier la partie distale (22) est disposé dans la tige (12).

14. Endoscope vidéo selon l'une des revendications 1 à 13, **caractérisé en ce que** le tube de poussée (80) et l'élément de traction (36) sont fixés à un boulon de traction (44), accouplé à l'élément de commande (32), sans pouvoir coulisser l'un par rapport à l'autre.

15. Endoscope vidéo selon l'une des revendications 1 à 14, **caractérisé en ce qu'**il est conçu sous la forme d'un endoscope vidéo d'intubation (10).
